# EUROPEAN PATENT SPECIFICATION

(11) **EP 3 643 324 B1**
(45) Date of publication and mention of the grant of the patent: **08.03.2023**
(21) Application number: 17915893.6
(22) Date of filing: 16.08.2017
(51) Int. Cl.: A61K 31/7034, A61K 31/352, A61P 27/10, A61P 27/02

(54) **SALIDROSIDE OR FORMONONETIN FOR INHIBITING MYOPIA**
SALIDROSID ODER FORMONONETIN ZUR HEMMUNG VON MYOPIE
SALIDROSIDE OU FORMONONETIN POUR L'INHIBITION DE LA MYOPIE

(30) Priority: 29.06.2017 CN 201710511445
(43) Date of publication of application: 29.04.2020
(73) Proprietor: Wenzhou Medical University, Wenzhou, Zhejiang 325000 (CN)
(72) Inventor: ZHOU, Xiangtian, Wenzhou Zhejiang 325000 (CN); QU, Jia, Wenzhou Zhejiang 325000 (CN); ZHAO, Fei, Wenzhou Zhejiang 325000 (CN); PAN, Miaozhen, Wenzhou Zhejiang 325000 (CN); ZHANG, Sen, Wenzhou Zhejiang 325000 (CN); ZHOU, Qingyi, Wenzhou Zhejiang 325000 (CN); WU, Hao, Wenzhou Zhejiang 325000 (CN)
(74) Representative: Viering, Jentschura & Partner mbB Patent- und Rechtsanwälte
(86) International application number: PCT/CN2017/097575
(87) International publication number: WO 2019/000605

(56) References cited:
- WO-A2-2007/016656
- JIANMING WU ET AL: "Formononetin, an active compound of <em>Astragalus membranaceus</em> (Fisch) Bunge, inhibits hypoxia-induced retinal neovascularization via the HIF-1[alpha]/VEGF signaling pathway", DRUG DESIGN, DEVELOPMENT AND THERAPY, vol. Volume 10, 23 September 2016 (2016-09-23), pages 3071-3081, XP055680505, DOI: 10.2147/DDDT.S114022
- ZHANG, XIAODI et al.: "Protective Effect of Salidroside on Rat Lung Injury Induced by Chlorine Gas Exposure", Journal of Preventive Medicine Information, vol. 29, no. 4, 30 April 2013 (2013-04-30) , pages 269-272, XP009518579, ISSN: 1006-4028
- GE, XIN et al.: "Formononetin Regresses HIF-la/CXCR4 Signaling and Cell Proliferation and Migration in Breast Cancer Cells", Henan Medical Research, vol. 25, no. 9, 1 September 2016 (2016-09-01), pages 1542-1545, XP055663352, DOI: 10.3969/j.issn.1004-437x.2016.09.003

## Description

### Technical Field

The invention relates to an inhibitor for myopia, particularly to an inhibitor of scleral hypoxia-inducible factor-1α for use in inhibiting myopia.

### Background Art

Currently, human myopia is a refractive error caused by over-extension of the ocular axis for clear retinal imaging, which is generally manifested as an over-extension of the ocular axis. The extension of the ocular axis is mainly manifested as the extension of the posterior pole. Through animal experiments, it was found that some pathological changes such as scleral thinning and posterior scleral expansion were found in myopic animal models such as *Gallus gallus domesticus, Tupαiα belangeri*, *Primates* and *Cavia porcellus.* The above evidence suggests that scleral tissue was remodeled during the occurrence and development of myopia.

Sclera consists of extracellular matrix and fibroblasts for secreting the matrix. Mammalian myopia model studies have found that the pathological changes in the sclera of myopic eyes are mainly due to changes in the amount of collagen, including decreased collagen synthesis and increased degradation.

### Technical Problem

The abnormal remodeling of the scleral collagen will change the biochemical properties of the sclera, making it thinner, which in turn will increase the scleral creep rate and reduce the tensile capacity. The thinned sclera cannot withstand normal intraocular pressure, which will cause the ocular axis to extend and form myopia. Therefore, the sclera is an important target for the occurrence and development of myopia.

WO 2007/016656 A2 relates to a method of treating or preventing an ocular disorder in a mammal mediated by hypoxia inducible factor-1 (HIF-I) that includes administering to the mammal a steroid that modulates the effects of local and systemic hypoxic events for the treatment of ocular disorders. Zhang, Xiaodi et al. ("Protective Effect of Salidroside on Rat Lung Injury induced by Chlorine Gas Exposure", Journal of Preventive Medicine Information, vol. 29, no. 4, April 30, 2013, pages 269-272, XP009518579) relates to the treatment of lung injury. Ge, Xin et al. ("Formononetin Regresses HIF-Ia/CXCR4 Signaling and Cell Proliferation and Migration in Breast Cancer Cells", Henan Medical Research, vol. 25, no. 9, September 1, 2016, pages 1542-1545, XP055663352) relates to the treatment of breast cancer. Jianming Wu et al. ("Formononetin, an active compound of emAstragalus membranaceus /em(Fisch) Bunge, inhibits hypoxia-induced retinal neovascularization via the HIF-1[alpha]/VEGF signalling pathway", drug design, development and therapy, vol. 10, September 23, 2016, pages 3071-3081, XP055680505) relates to the treatment of hypoxia-induced retinal neovascularization.

### Solution to the Problem

### Technical Solution

In order to solve the deficiencies of the prior art, a single-cell transcriptome analysis was performed in the present invention and the result found that eIF2α and Nrf2 pathways related to tissue hypoxia were abnormally activated in myofibroblasts, suggesting that tissue hypoxia may play an important role in the scleral extracellular matrix remodeling. We have further found that myopic induction may specifically cause the expression of scleral hypoxia-inducible factor 1α (HIF1 α) to be up-regulated, and return to normal during the recovery period of myopia, which indicates that the scleral tissue is in a state of hypoxia when myopia occurred, and hypoxia also is a key factor in inducing myopia formation. In the disclosure, a method for inhibiting myopia and an application in preparing a drug are provided; myopia is interfered by inhibiting the expression of HIF-1α and inhibiting intraocular hypoxia, and a method for inhibiting myopia is found.

The technical solution adopted by the invention is as follows: an inhibitor for myopia by inhibiting intraocular scleral hypoxia.

The inhibitor inhibits intraocular scleral hypoxia by inhibiting the expression of scleral hypoxia-inducible factor-1α (HIF-1α), thereby inhibiting myopia.

An application of an inhibitor of scleral hypoxia-inducible factor-1α in preparing a drug for inhibiting myopia is disclosed.

The inhibitor of scleral hypoxia-inducible factor-1α is salidroside or formononetin.

An application of salidroside or formononetin in preparing a drug for inhibiting intraocular scleral hypoxia is disclosed.

### Advantageous Effects of the Invention

### Advantageous Effects

The invention has the advantageous effects that the invention provides an inhibitor for myopia according to claim 1. Anti-hypoxia is achieved by inhibiting the expression of intraocular HIF-1α, so that myopia is effectively delayed and inhibited. The present invention utilizes HIF-1α inhibitors salidroside and formononetin to inhibit HIF-1α activity so as to play an anti-hypoxia effect and obviously play a role in delaying myopia.

### Brief Description for the Drawings

### Brief Description of the Drawings

Fig. 1 shows the expression of HIF-1α protein in the sclera of normal control eyes, 1 week form-deprivation experimental eyes and contralateral eyes, and 1 week form-deprivation & 2-day recovery experimental eyes and contralateral eyes.
Fig. 2 is a graph of diopter difference between the experimental eyes injected with salidroside (SDS) and contralateral eyes.
Fig. 3 is a graph of vitreous chamber depth difference between the experimental eyes injected with salidroside (SDS) and contralateral eyes.
Fig. 4 is a graph of ocular axis length difference between the experimental eyes injected with salidroside (SDS)
Fig. 5 is a graph of diopter difference between the experimental eyes injected with formononetin (FMN) and contralateral eyes.
Fig. 6 is a graph of ocular axis length difference between the experimental eyes injected with formononetin (FMN) and contralateral eyes.
Fig. 7 shows changes in scleral HIF-1α and collagen expression after injecting with salidroside (SDS).
Fig. 8 shows changes in scleral HIF-1α and collagen expression after injecting formononetin (FMN).

In the figures, "difference" refers to the difference in diopter or ocular axis parameters between the experimental eyes and contralateral eyes; a comparison between the salidroside injection solvent group and the administration group was performed using two-way analysis of variance (ANOVA) with repeated measurements: "*" means P<0.05, "**" means P<0.01, and "***" means P<0.001. A comparison between the formononetin injection solvent group and the administration group was performed using one-way analysis of variance (ANOVA): "*" means P<0.05.

### Embodiments of the Invention

### Detailed Description of the Invention

The experimental animals used in this experiment were 3-week-old British shorthair three-colored guinea pigs (*Cavia porcellus).* Mask technique was used as a model of monocular form-deprivation (FD) myopia. In the myopic sclera HIF-1α expression experiment, the animals were randomly divided into three groups: the normal control group, FD 1 week group, and FD 1 week & 2-day recovery group. After the model was constructed, the sclera was removed, and the expression of HIF-1α in the sclera was detected by Western Blot, indicating that the sclera was in a state of hypoxia; however, the expression of HIF-1α restored to normal level during convalescence, indicating that hypoxia was closely related to myopia.

In the administration experiment, HIF-1α inhibitors salidroside and formononetin were administered to the form-deprived eyes by periocular injection to inhibit HIF-1α activity. The animals in the salidroside injection experiment were randomly divided into three groups: form-deprivation+solvent control group (FD+0.9% normal saline (NS)), form-deprivation+low concentration drug group (FD+SDS 1 µg), and form-deprivation+high concentration drug group (FD+SDS 10 µg). The animals in the formononetin injection experiment were randomly divided into three groups: form-deprivation+solvent control group (FD+DMSO), form-deprivation+low concentration drug group (FD+FMN 0.5 µg), and form-deprivation+high concentration drug group (FD+FMN 5 µg). Periocular injection of drugs was performed daily at 9 am for 4 weeks without treatment of the contralateral eyes. Before the experiment and after 2 weeks and 4 weeks of administration respectively, the diopter was measured with an eccentric infrared photo-refractor (EIR), and the ocular axis parameters such as vitreous chamber depth and ocular axis length were measured with amplitude-mode ultrasound (11 MHz). After the administration, the sclera was removed and the expression of HIF-1α and collagen type I were detected by Western Blot. Detection of HIF-1α in the sclera of myopic eyes showed the expression level of HIF-1α in FD 1 week experimental eyes (FD T) was significantly higher than that in contralateral eyes (FD F). After 2 days of recovery in FD 1 week experimental eyes (RC), the difference in the expression of HIF-1α in the eyes disappeared. It is suggested that the scleral tissue was in a state of hypoxia when myopia occurred, and hypoxia may be involved in regulating the occurrence and development of myopia.

By comparing the measured parameters before and after the experiment, it was found that the degree of refractive myopia and the degrees of extension of vitreous chamber and ocular axis in the form-deprived eyes in the administration groups were less than those in the form-deprived solvent groups, which had statistical significance compared with the solvent control groups. Therefore, the inhibition of HIF-1α activity by peribulbar injection of salidroside and formononetin may inhibit the formation of form-deprivation myopia in *Cavia porcellus.*

After the administration, the expression levels of HIF-1α and collagen type I were detected. It was found that the up-regulated expression of HIF-1α and the down-regulated expression of collagen type I were inhibited after peribulbar injection of salidroside and formononetin. Therefore, the expression of collagen type I in the sclera may be regulated by peribulbar injection of HIF-1α inhibitors salidroside and formononetin.

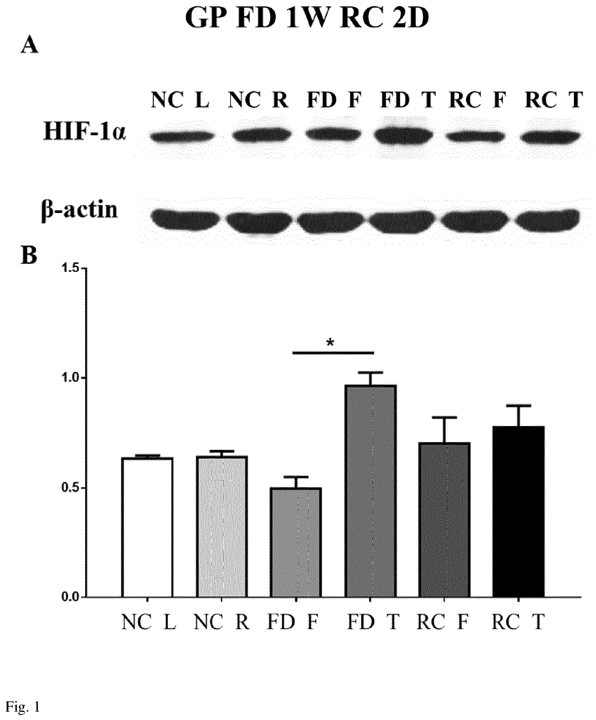

As can be seen from Fig. 1, the expression of HIF-1α in the sclera of the form-deprived eyes was significantly up-regulated compared with that in the contralateral eyes after 1 week of form-deprivation, but there was no difference in the expression of HIF-1α in the two eyes of form-deprivation & 2-day recovery group, indicating that the scleral tissue was in a state of hypoxia when myopia occurred.

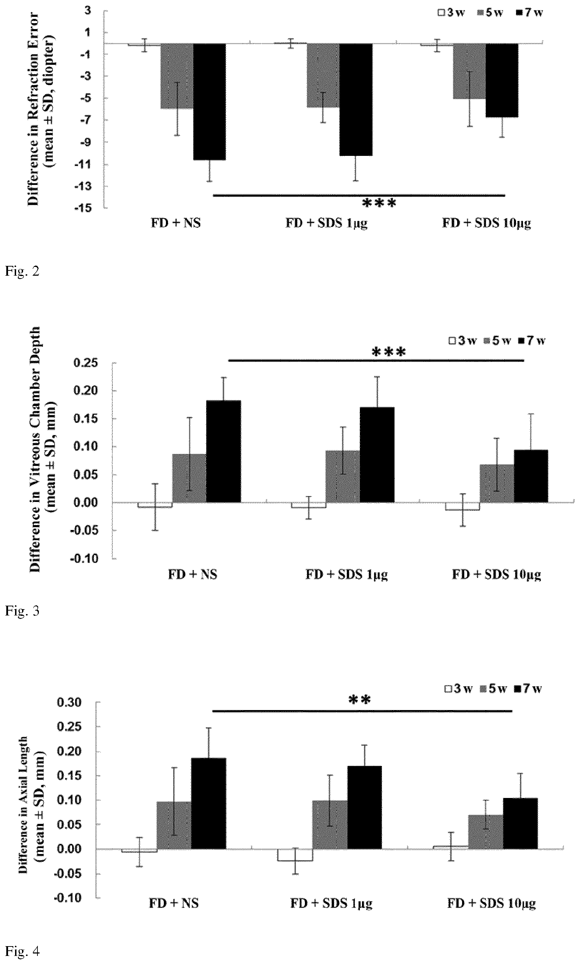

As can be seen from Fig. 2, after 4 weeks of the experiment, the myopia formation in the form-deprivation salidroside-injection group was significantly reduced compared with that of the form-deprivation solvent-injection group, indicating that the HIF-1α inhibitor salidroside may inhibit the progression of form-deprivation myopia.

As can be seen from Fig. 3, after 4 weeks of the experiment, the extension of vitreous chamber in the salidroside-injection group was relatively smaller than that of the solvent-injection group, indicating that the HIF-1α inhibitor salidroside may inhibit the extension of the form-deprived vitreous chamber.

As can be seen from Fig. 4, after 4 weeks of the experiment, the extension of ocular axis in the salidroside-injection group was relatively smaller than that of the solvent-injection group, indicating that the HIF-1α inhibitor salidroside may inhibit the extension of the form-deprived ocular axis.

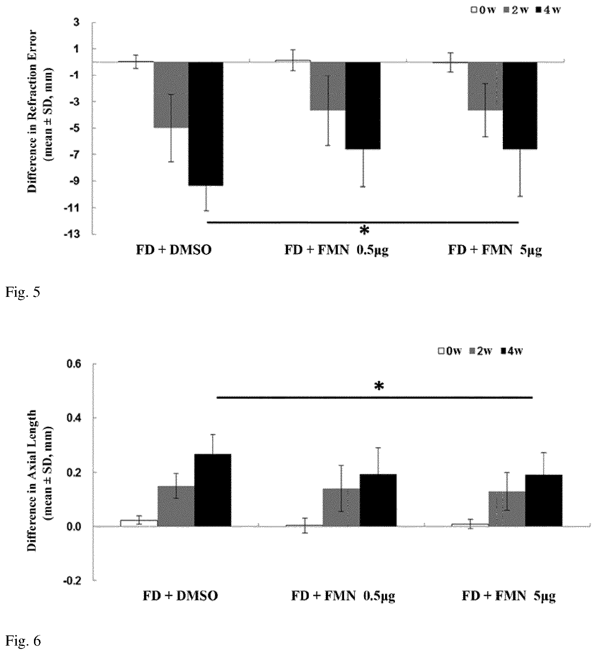

As can be seen from Fig. 5, after 4 weeks of the experiment, the myopia formation in the form-deprivation formononetin-injection group was significantly reduced compared with that of the form-deprivation solvent-injection group, indicating that the HIF-1α inhibitor formononetin may inhibit the progression of form-deprivation myopia.

As can be seen from Fig. 6, after 4 weeks of the experiment, the extension of the ocular axis in the formononetin-injection group was relatively smaller than that in the solvent-injection group, indicating that the HIF-1α inhibitor formononetin may inhibit the extension of the form-deprived ocular axis.

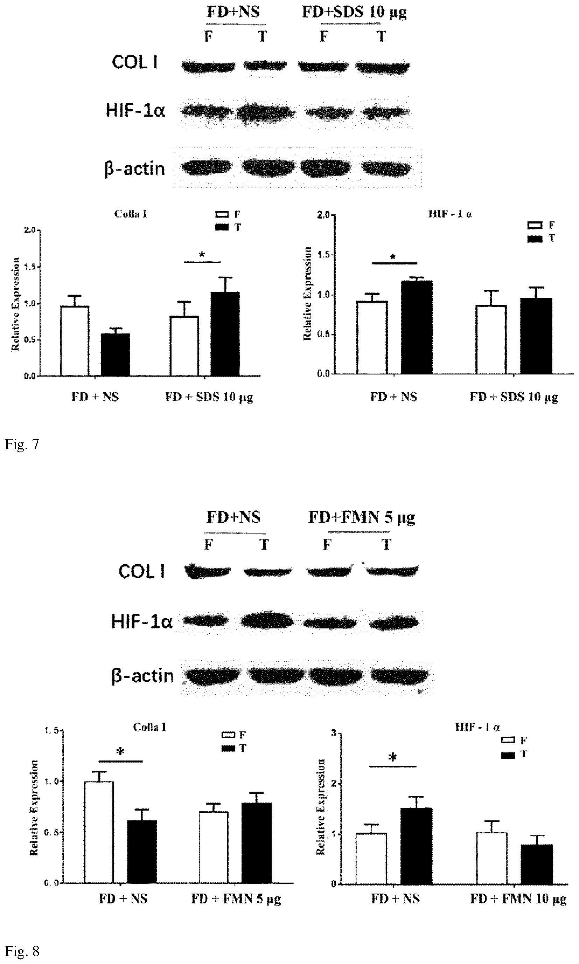

As can be seen from Fig. 7, after salidroside injection, the up-regulation of HIF-1α and down-regulation of collagen expression in the sclera of FDM eyes were inhibited, indicating that the HIF-1α inhibitor salidroside may inhibit the progression of myopia by regulating collagen type I in the sclera.

As can be seen from Fig. 8, after formononetin injection, the up-regulation of HIF-1α and down-regulation of collagen expression in the sclera of FDM eyes were inhibited, indicating that HIF-1α inhibitor formononetin may inhibit the progression of myopia by regulating collagen type I in the sclera.

The above experiments proved that the sclera tissue was in a state of hypoxia when myopia occurred. The HIF-1α inhibitors salidroside and formononetin were used for inhibiting the HIF-1α activity to play an anti-hypoxia role and obviously play a role in delaying myopia. The inhibitory effects of salidroside and formononetin on myopia may be achieved by regulating collagen type I in the sclera. The above-mentioned embodiments are merely preferred embodiments of the present invention, and the scope of the present invention is not limited to the above-mentioned embodiments. It should be noted that those skilled in the art will appreciate that various modifications and adaptations can be made without departing from the scope of the present invention.

## Claims

1. Inhibitor of scleral hypoxia-inducible factor-1α for use in inhibiting myopia,
wherein the inhibitor of scleral hypoxia-inducible factor-1α is salidroside or formononetin.

## Patentansprüche

1. Inhibitor des skleralen hypoxie-induzierbaren Faktors-1α zur Verwendung beim Hemmen von Myopie,
wobei der Inhibitor des skleralen hypoxie-induzierbaren Faktors-1α Salidrosid oder Formononetin ist.

## Revendications

1. Le facteur scléral inhibiteur induit par l'hypoxie-la pour inhiber la myopie,
ou le facteur scléral inhibiteur induit par l'hypoxie-1α; est le salidroside ou la formononétine.
